Europäisches Patentamt

European Patent Office

Office européen des brevets

(11) Veröffentlichungsnummer: **0 303 062 B1**

(12) **EUROPÄISCHE PATENTSCHRIFT**

(45) Veröffentlichungstag der Patentschrift: **06.05.92**

(21) Anmeldenummer: **88111270.0**

(22) Anmeldetag: **14.07.88**

(51) Int. Cl.⁵: **C12N 9/96**, C12N 9/08, G01N 33/58

(54) Stabilisiertes Peroxidasepräparat.

(30) Priorität: **11.08.87 DE 3726634**

(43) Veröffentlichungstag der Anmeldung:
**15.02.89 Patentblatt 89/07**

(45) Bekanntmachung des Hinweises auf die
Patenterteilung:
**06.05.92 Patentblatt 92/19**

(84) Benannte Vertragsstaaten:
**AT BE CH DE ES FR GB GR IT LI LU NL SE**

(56) Entgegenhaltungen:
EP-A- 0 044 432      EP-A- 0 065 800
EP-A- 0 152 847      WO-A-86/04095
DE-A- 1 492 060      US-A- 4 252 896

CHEMICAL ABSTRACTS, Band 101, Nr. 1, 2.
Juli 1984, Seite 269, Zusammenfassung nr.
3053j, Columbus, Ohio, US; & JP-A-59 25 683
(TOYOBA CO., LTD) 09-02-1984

(73) Patentinhaber: **Biotest AG**
**Flughafenstrasse 4**
**W-6000 Frankfurt am Main 73(DE)**

(72) Erfinder: **Horn, Jürgen, Dr. Dipl.-Chem.**
**Kurt-Schumacher-Ring 83**
**W-6073 Egelsbach(DE)**

(74) Vertreter: **Beil, Hans Chr., Dr. et al**
**Beil, Wolff und Beil, Rechtsanwälte Adelon-**
**strasse 58 Postfach 80 01 40**
**W-6230 Frankfurt am Main 80(DE)**

**Beschreibung**

Die Erfindung betrifft ein stabilisiertes Peroxidasepräparat.

Peroxidasen werden für verschiedenste Zwecke verwendet, darunter auch als nachweisbare Marker bei immunologischen Verfahren zum Nachweis und zur Bestimmung von Immunreaktanten wie Haptenen, Antigenen oder Antikörpern. Die Verwendung von mit Peroxidase markierten Immunreaktanten ist von besonderem Interesse, da die Aktivität oder Anwesenheit des Enzyms optisch nachweisbar ist und der Aktivitätsgrad durch kolorimetrische Verfahren bestimmt werden kann.

Testpackungen zur Durchführung von enzymatischen Immuntests enthalten im allgemeinen als wesentlichen Bestandteil eine bestimmte Menge eines an eine Peroxidase gekuppelten Immunreaktanten. Da derartige Testpackungen versandt und vor ihrer Verwendung unterschiedlich lang gelagert werden müssen, ist es wichtig, daß die Enzymaktivität möglichst lang erhalten bleibt.

Es ist bekannt, daß Peroxidasen in Lösung, besonders in niedrigen Konzentrationen, nicht sehr stabil sind, unabhängig davon, ob sie an einen anderen Bestandteil chemisch gebunden (konjugiert) sind oder nicht. Infolgedessen ist ihre Lagerstabilität gering, was zu kurzen Laufzeiten bei Verwendung in Enzymimmuntests führt. Auch ist es schwierig, die Testpackungen ohne Kühlung zu verschicken, da beim Versand im Sommer für einige Stunden stark erhöhte Temperaturen auftreten können.

Man war deshalb bestrebt, Peroxidase bzw. Peroxidasepräparate zu stabilisieren.

In der DE-PS 31 00 076 wird der Zusatz von 0,01 % 8-Anilino-1-naphthalinsulfonsäure beschrieben, um Peroxidasekonjugate in serumproteinhaltiger Lösung zu stabilisieren.

In der DE-OS 35 11 327 wird der Zusatz von 0,0005 bis 2 Gew.-% Aminopyrin beschrieben, der zu einer Stabilisierung bei 30°C führt.

In der EP-A 01 52 847 wird der Zusatz von Calciumsalzen und Polyethylenglykol beschrieben, welches zu einer Stabilisierung bei 37°C und 45°C führt.

Wegen der Versandbedingungen ist eine Stabilisierung bei 37°C oder besser 45°C erwünscht, eine Stabilisierung bei 30°C ist hierfür unzureichend. Gleichzeitig sollte das Stabilisierungsreagenz jedoch eine Zunahme des Backgrounds im Test, d.h. das unspezifische Binden von Enzymkonjugat an die Festphase, nicht erhöhen.

Diese Bedingungen werden durch die bekannten Stabilisatoren teilweise nicht bzw. nur unzureichend erfüllt.

Der Erfindung liegt die Aufgabe zugrunde, ein stabilisiertes Peroxidasepräparat bereitzustellen, das bei relativ hohen Temperaturen über einen längeren Zeitraum stabil ist, dessen Stabilisierungsmittel ein unspezifisches Binden von Peroxidase bzw. Peroxidasekonjugat an die Festphase bei einem Immuntest nicht oder nicht wesentlich erhöht und das auch die übrigen Nachteile bisheriger stabilisierter Peroxidasepräparate nicht oder in wesentlich geringerem Maße aufweist.

Diese Aufgabe wird erfindungsgemäß dadurch gelöst, daß das Peroxidasepräparat als Stabilisator hydrolysiertes Hühneralbumin oder eine Kombination aus Kalbsserum und Polyvinylpyrrolidon und/oder Calciumpropionat oder Gemische davon enthält.

Bekanntlich führt die Verwendung von humanem Albumin oder bovinem Serumalbumin in höheren Konzentrationen wie 0,5-1% zu einer starken Inaktivierung der Peroxidase, vermutlich durch Häminwechselwirkung zwischen der Peroxidase und dem Hämin bindenden Albumin.

Es war deshalb überraschend und völlig unerwartet, daß hydrolysiertes Hühneralbumin nicht nur nicht zu einer Inaktivierung der Peroxidase führt, sondern im Gegenteil, eine ausgesprochen gute Stabilisierung der Peroxidase bewirkt.

Daß bei Kombination mit 8-Anilino-1-naphthalinsulfonsäure die bekannte Inaktivierungswirkung von Kalbsserum gegenüber Peroxidase bis zu einem gewissen Grad herabgesetzt werden kann, ist zwar aus der vorstehend genannten DE-PS 31 00 076 bekannt, jedoch war es überraschend, daß durch die beanspruchte Kombination von Kalbsserum mit Polyvinylpyrrolidon und/oder Calciumpropionat wesentlich bessere Stabilisierungseffekte erzielt werden können.

Die Wirkung der beanspruchten Stabilisierungsmittel kann durch Zugabe eines wasserlöslichen Salzes der 8-Anilino-1-naphthalinsulfonsäure in einigen Fällen noch verbessert werden. Dabei stellte man fest, daß die Stabilisierungswirkung der Salze gegenüber der an sich bekannten Wirkung der Säure wesentlich besser ist. Zu den wasserlöslichen Salzen gehören z.B. die Ammonium-, Na-, K-, Li-, Mg- oder Ca-Salze. Besonders bevorzugt wird das Ammoniumsalz.

Die Menge der Stabilisatoren beträgt etwa 0,1 bis 20 Gew.-%, bezogen auf die Lösung des Peroxidasepräparates. Dabei kann sich diese Menge auf jeden einzelnen Stabilisator oder auf ein Gemisch derselben beziehen. Die Gesamtmenge an Stabilisatoren sollte jedoch 20 Gew.-% nicht übersteigen.

Im Falle des hydrolysierten Hühneralbumins beträgt die bevorzugte Konzentration 0,5 bis 3 Gew.-%, bezogen auf die Lösung, besonders bevorzugt 1 bis 2 Gew.-%.

Die bevorzugte Menge des Polyvinylpyrrolidons beträgt etwa 0,5 bis 2 Gew.-%, bezogen auf die Lösung, besonders bevorzugt 1 Gew.-%. Das Molekulargewicht desselben beträgt zweckmäßigerweise 5 000 bis 20 000, vorzugsweise 10 000.

Die bevorzugte Menge des Calciumpropionats beträgt etwa 0,1 bis 1 Gew.-%, bezogen auf die Lösung, besonders bevorzugt 0,2 Gew.-%.

Die Konzentration des Kalbsserums kann 10 bis 75 Gew.-%, bezogen auf die Lösung, betragen, vorzugsweise etwa 50 Gew.-%.

Die Mengen der fakultativen Zusätze können beim wasserlöslichen Salz der 8-Anilino-1-naphthalinsulfonsäure etwa 0,1 bis 1 Gew.-% und beim Methoxypolyethylenglycol etwa 0,1 bis 10 Gew.-%, vorzugsweise 1 Gew.-%, bezogen auf die Lösung, betragen.

Zu den Lösungsmedien können beispielsweise sämtliche Seren gehören, wie beispielsweise Ziegen-, Pferd- und Meerschweinchenserum sowie wäßrige Pufferlösungen.

Die Peroxidasekonjugate umfassen Antigen- und Antikörperkonjugate. Als Antikörper kann jeder beliebige polyklonale oder monoklonale Antikörper dienen.

Nachstehende Beispiele dienen der weiteren Erläuterung der Erfindung.

Beispiel 1

Hydrolysiertes Hühneralbumin (Albumin chicken egg hydrolysate, Sigma) wurde mit 0,01M TRIS vom pH-Wert 7,2 mit einem Gehalt an 0,15M NaCl auf eine Endkonzentration von 2 Gew.-% Hühneralbumin verdünnt. Danach wurden 0,3 µg Peroxidasekonjugat pro ml dieser Lösung zugesetzt.

Beispiel 2

Handelsübliches Kalbsserum wurde 1 Stunde bei 55°C inaktiviert und anschließend mit hydrolysiertem Hühneralbumin, TRIS-Puffer und Peroxidasekonjugat wie in Beispiel 1 behandelt unter Erzielung einer Endkonzentration von 2 Gew.-% Hühneralbumin und 50 Gew.-% Kalbsserum.

Beispiel 3

Man verfuhr nach Beispiel 2 mit dem Unterschied, daß man das inaktivierte Kalbsserum vor der Zugabe der übrigen Substanzen 16 Stunden mit dem Ammoniumsalz der 8-Anilino-1-naphthalinsulfonsäure behandelte in einer Menge, daß die Endkonzentration des Ammoniumsalzes in der Lösung 1 Gew.-% betrug.

Beispiel 4

Man verfuhr nach Beispiel 3 mit dem Unterschied, daß man anstelle von hydrolysiertem Huhneralbumin Polyvinylpyrrolidon mit einem Molekulargewicht von 10 000 verwendete in einer Menge, daß die Endkonzentration des Pyrrolidons in der Lösung 1 Gew.-% betrug.

Beispiel 5

Man verfuhr nach Beispiel 4 mit dem Unterschied, daß man zusätzlich Calciumpropionat in einer Menge zusetzte, daß dessen Endkonzentration in der Lösung 0,2 Gew.-% betrug.

Beispiel 6

Man verfuhr nach Beispiel 5 mit dem Unterschied, daß man zusätzlich noch hydrolysiertes Hühneralbumin zusetzte in einer Menge, daß dessen Endkonzentration in der Lösung 1 Gew.-% betrug.

Beispiel 7

Man verfuhr nach Beispiel 2 mit dem Unterschied, daß man anstelle von hydrolysiertem Hühneralbumin Polyvinylpyrrolidon mit einem Molekulargewicht von 10 000 und Calciumpropionat zusetzte in einer Menge, daß deren Endkonzentrationen in der Lösung 1 Gew.-% bzw. 0,2 Gew.-% betrugen.

Beispiel 8

Man verfuhr nach Beispiel 2 mit dem Unterschied, daß man zusätzlich Polyvinylpyrrolidon mit einem Molekulargewicht von 10 000 und Calciumpropionat zusetzte unter Erzielung der in Beispiel 7 angegebenen Endkonzentrationen derselben in der Lösung.

Beispiel 9

Man verfuhr nach Beispiel 3 mit dem Unterschied, daß man anstelle von hydrolysiertem Hühneralbumin Methoxyethylenglycol und Calciumpropionat zusetzte in einer Menge, daß deren Endkonzentrationen in der Losung 1 Gew.-% bzw. 0,2 Gew.-% betrugen.

Beispiel 10

Man verfuhr nach Beispiel 7 mit dem Unterschied, daß man zusätzlich Methoxypolyethylenglycol und Calciumpropionat zusetzte unter Erzielung der in Beispiel 9 angegebenen Endkonzentrationen derselben in der Lösung.

Zum Austesten der Stabilität der nach den Beispielen 1 bis 10 hergestellten Peroxidasekonjugat-Präparate wurde ein Enzymimmuntest auf Cytomegalovirus (CMV) IgG-Antikörper durchgeführt. Verwendet wurden CMV-Antigen und Kontrollantigen beschichtete Mikrotiterplatten. Das Kontrollantigen bestand aus Antigen aus nicht CMV-infizierten Fibroblasten ohne CMV-Antigen. Es wurde mit anti-CMV-positiven Proben 30 Min. bei $40°C$ inkubiert, gewaschen, dann mit den Peroxidaseantikörperkonjugaten 30 Min. bei $40°C$ inkubiert, gewaschen, mit $H_2O_2$/Orthophenylendiamin als Peroxidasesubstrat 10 Min. bei Raumtemperatur inkubiert, mit $H_2SO_4$ abgestoppt und die optische Dichte im Photometer gemessen.

Als Peroxidase markierte Antikörper wurden Ziege anti human IgG (Fc-Teil spezifisch) und ein Maus monoklonaler Antikorper gegen human IgG (BS 12 IgG spezifisch) verwendet. Die Markierung der Antikörper erfolgte durch Perjodat-Oxidation von Peroxidase mit Entfernung des freien Perjodats und anschließender Kupplung an die verwendeten Antikörper (vgl. The Journal of Histochemistry and Cytochemistry, Vol. 22, No. 12, pp.1084-1091 (1974)).

Die Testergebnisse sind aus den anliegenden Tabellen I und II ersichtlich.
Darin bedeutet OD = optische Dichte. In Spalte 1 eines jeweiligen Versuchs sind die Werte für das CMV-Antigen angegeben, in Spalte 2 die Werte für das Kontrollantigen, und Spalte 3 zeigt die Differenz zwischen Spalte 1 und 2 an.
Der Meßbereich des Photometers geht nur bis OD = 1,5, so da8 bei einigen Messungen keine Differenzwerte angegeben werden konnten.

Ein Differenzwert zwischen 0h (Stunde Null) und Xh (Stunde X) von über 0,2 gilt als negatives Ergebnis, d.h. keine ausreichende Stabilität, so daß, wenn dieser Wert an irgendeinem Testzeitpunkt überschritten ist, kein weiterer Test erfolgt (siehe z.B. Tabelle II, Vergleichsversuch mit Kalbsserum ohne Stabilisatorzusätze 48 h).

Die festgestellte absolute Extinktion mit dem Kontrollantigen ist ein Maß für die jeweilige Änderung (Erhöhung) der Backgroundaktivität. Die entsprechenden Versuche werden im Vergleich zu nicht stabilisierten Peroxidasekonjugatlösungen durchgeführt.

Eingesetzt wurden in allen Vergleichstests identische Chargen des gleichen Konjugats in gleicher Konzentration. Für den endgültigen Test können unabhängig davon abweichende Konjugatkonzentrationen von 0,03 bis 3 mg Enzymkonjugat pro Liter des stabilisierenden Verdünnungsmediums verwendet werden.

Praktisch gleiche Ergebnisse erhält man, wenn man in den Beispielen anstelle des TRIS-Puffers PBS-Puffer verwendet.

Tabelle I

Ziege anti human IgG POD markiert

| Art des Verdünnungsmediums | 0h 37° OD CMV Ag | 0h 37° OD Kontr. Ag | 0h 37° ∆OD CMV Ag Ko. A | 24h 37° OD CMV Ag | 24h 37° OD Kontr. Ag | 24h 37° ∆OD CMV Ag Ko. Ag | 48h 37° OD CMV Ag | 48h 37° OD Kontr. Ag | 48h 37° ∆OD CMV Ag Ko. Ag | 72h 37° OD CMV Ag | 72h 37° OD Kontr. Ag | 72h 37° ∆OD CMV Ag Ko. Ag | Test-konzentration anti CMV IgG |
|---|---|---|---|---|---|---|---|---|---|---|---|---|---|
| 1 | 0,758 | 0,192 | 0,566 | 0,736 | 0,182 | 0,544 | 0,695 | 0,171 | 0,514 | 0,626 | 0,165 | 0,461 | 0,4 U/ml |
|  | 1,112 | 0,187 | 0,925 | 1,087 | 0,123 | 0,904 | 0,952 | 0,174 | 0,778 | 0,936 | 0,168 | 0,768 | 0,8 U/ml |
| 2 | 0,694 | 0,061 | 0,633 | 0,685 | 0,059 | 0,626 | 0,637 | 0,053 | 0,584 | 0,642 | 0,055 | 0,587 | 0,4 U/ml |
|  | 1,028 | 0,064 | 0,964 | 0,997 | 0,058 | 0,939 | 0,993 | 0,054 | 0,939 | 0,081 | 0,051 | 0,930 | 0,8 U/ml |
| 3 | 0,523 | 0,067 | 0,453 | 0,502 | 0,059 | 0,443 | 0,498 | 0,055 | 0,443 | 0,481 | 0,052 | 0,429 | 0,4 U/ml |
|  | 0,837 | 0,062 | 0,775 | 0,797 | 0,058 | 0,741 | 0,751 | 0,053 | 0,698 | 0,735 | 0,054 | 0,681 | 0,8 U/ml |
|  | >1,5 | 0,063 |  | >1,5 | 0,060 |  | >1,5 | 0,052 |  | >1,5 | 0,050 |  | 4,5 U/ml |
| 4 | 0,725 | 0,053 | 0,672 | 0,707 | 0,049 | 0,658 | 0,682 | 0,046 | 0,636 | 0,693 | 0,044 | 0,649 | 0,4 U/ml |
|  | 1,086 | 0,057 | 1,029 | 1,036 | 0,048 | 0,988 | 0,993 | 0,049 | 0,954 | 1,021 | 0,051 | 0,970 | 0,8 U/ml |
|  | >1,5 | 0,058 |  | >1,5 | 0,051 |  | >1,5 | 0,048 |  | >1,5 | 0,047 |  | 4,5 U/ml |
| 5 | 0,588 | 0,062 | 0,526 | 0,526 | 0,053 | 0,473 | 0,517 | 0,048 | 0,469 | 0,493 | 0,047 | 0,446 | 0,4 U/ml |
|  | 0,856 | 0,053 | 0,803 | 0,764 | 0,051 | 0,713 | 0,731 | 0,046 | 0,685 | 0,702 | 0,049 | 0,653 | 0,8 U/ml |
| 6 | 0,573 | 0,054 | 0,519 | 0,549 | 0,052 | 0,489 | 0,528 | 0,051 | 0,477 | 0,505 | 0,045 | 0,460 | 0,4 U/ml |
|  | 0,827 | 0,056 | 0,771 | 0,775 | 0,049 | 0,726 | 0,748 | 0,047 | 0,701 | 0,729 | 0,038 | 0,691 | 0,8 U/ml |
| 7 | 0,718 | 0,039 | 0,679 | 0,695 | 0,037 | 0,658 | 0,708 | 0,041 | 0,667 | 0,691 | 0,039 | 0,652 | 0,4 U/ml |
|  | 1,054 | 0,038 | 1,016 | 1,023 | 0,042 | 0,981 | 1,005 | 0,043 | 0,962 | 0,984 | 0,036 | 0,948 | 0,8 U/ml |
|  | >1,5 | 0,043 |  | >1,5 | 0,044 |  | >1,5 | 0,040 |  | >1,5 | 0,035 |  | 4,5 U/ml |
| 8 | 0,573 | 0,052 | 0,521 | 0,557 | 0,046 | 0,511 | 0,538 | 0,039 | 0,499 | 0,519 | 0,042 | 0,477 | 0,4 U/ml |
|  | 0,816 | 0,048 | 0,768 | 0,798 | 0,048 | 0,750 | 0,781 | 0,044 | 0,737 | 0,772 | 0,038 | 0,734 | 0,8 U/ml |
|  | >1,5 | 0,047 |  | >1,5 | 0,051 |  | >1,5 | 0,055 |  | >1,5 | 0,047 |  | 4,5 U/ml |

Tabelle I  (Fortsetzung)

| Art des Verdünn-ungsmediums | Oh 37° OD CMV Ag | OD Kontr. Ag | ΔOD CMV Ag Ko. A | 24h 37° OD CMV Ag | OD Kontr. Ag | ΔOD CMV Ag Ko. Ag | 48h 37° OD CMV Ag | OD Kontr. Ag | ΔOD CMV Ag Ko. Ag | 72h 37° OD CMV Ag | OD Kontr. Ag | ΔOD CMV Ag Ko. Ag | Test-konzentra-tion anti CMV IgG |
|---|---|---|---|---|---|---|---|---|---|---|---|---|---|
| 9 | 0,599 | 0,046 | 0,553 | 0,584 | 0,050 | 0,534 | 0,561 | 0,041 | 0,520 | 0,558 | 0,037 | 0,521 | 0,4 U/ml |
|  | 0,922 | 0,044 | 0,878 | 0,938 | 0,039 | 0,899 | 0,898 | 0,036 | 0,862 | 0,902 | 0,039 | 0,863 | 0,8 U/ml |
|  | >1,5 | 0,046 |  | >1,5 | 0,041 |  | >1,5 | 0,042 |  | >1,5 | 0,035 |  | 4,5 U/ml |
| 10 | 0,630 | 0,138 | 0,492 | 0,621 | 0,127 | 0,494 | 0,603 | 0,119 | 0,484 | 0,571 | 0,105 | 0,465 | 0,4 U/ml |
|  | 1,023 | 0,134 | 0,899 | 0,984 | 0,131 | 0,853 | 0,917 | 0,108 | 0,809 | 0,853 | 0,102 | 0,751 | 0,8 U/ml |
| 11 | 0,675 | 0,129 | 0,546 | 0,652 | 0,119 | 0,533 | 0,609 | 0,110 | 0,499 | 0,537 | 0,098 | 0,439 | 0,4 U/ml |
|  | 1,021 | 0,121 | 0,900 | 0,965 | 0,123 | 0,842 | 0,932 | 0,112 | 0,820 | 0,881 | 0,100 | 0,781 | 0,8 U/ml |
| keine Stabilisa-torenzusätze Kalbsserum in-aktiviert als 50% ige Lösung in 0,01M TRIS ge-pufferter Koch-salzlsg pH 7.2 | 0,485 | 0,069 | 0,416 | B,197 | 0,037 | 0,160 | 0,098 | 0,021 | 0,077 | 0,065 | 0,014 | 0,051 | 0,4 U/ml |
|  | 0,805 | 0,072 | 0,773 | 0,308 | 0,032 | 0,274 | 0,185 | 0,027 | 0,158 | 0,137 | 0,020 | 0,117 | 0,8 U/ml |
|  | >1,5 | 0,065 |  | 0,855 | 0,038 | 0,817 | 0,483 | 0,019 | 0,464 | 0,335 | 0,017 | 0,318 | 4,5 U/ml |

EP 0 303 062 B1

EP 0 303 062 B1

Tabelle II

Maus anti human IgG (monoklonal) POD

| Art des Verdünn- ungsmediums | OD CMV Ag | Oh 50° OD Kontr. Ag | ΔOD CMV Ag Ko. A | OD CMV Ag | 24h 50° OD Kontr. Ag | ΔOD CMV Ag Ko. Ag | OD CMV Ag | 48h 50° OD Kontr. Ag | ΔOD CMV Ag Ko. Ag | OD CMV Ag | 72h 50° OD Kontr. Ag | ΔOD CMV Ag Ko. Ag | Test- konzentra- tion anti CMV IgG |
|---|---|---|---|---|---|---|---|---|---|---|---|---|---|
| 1 | 0,553 | 0,063 | 0,490 | 0,518 | 0,060 | 0,458 | 0,477 | 0,061 | 0,416 | 0,413 | 0,052 | 0,361 | 0,4 U/ml |
|   | 0,879 | 0,067 | 0,812 | 0,818 | 0,055 | 0,763 | 0,752 | 0,058 | 0,694 | 0,702 | 0,051 | 0,651 | 0,8 U/ml |
| 2 | 0,489 | 0,002 | 0,487 | 0,453 | 0,003 | 0,450 | 0,428 | 0,007 | 0,421 | 0,389 | 0,004 | 0,385 | 0,4 U/ml |
|   | 0,778 | 0,005 | 0,773 | 0,705 | 0,004 | 0,701 | 0,629 | 0,003 | 0,626 | 0,578 | 0,005 | 0,573 | 0,8 U/ml |
| 3 | 0,445 | 0,006 | 0,439 | 0,398 | 0,001 | 0,397 | 0,365 | 0,003 | 0,362 | 0,321 | 0,002 | 0,319 | 0,4 U/ml |
|   | 0,718 | 0,008 | 0,710 | 0,635 | 0,005 | 0,630 | 0,571 | 0,004 | 0,567 | 0,516 | 0,001 | 0,515 | 0,8 U/ml |
|   | >1,5 | 0,009 |   | >1,5 | 0,006 |   | 1,445 | 0,007 | 1,438 | 1,316 | 0,006 | 1,310 | 4,5 U/ml |
| 5 | 0,576 | 0,002 | 0,574 | 0,522 | 0,003 | 0,519 | 0,451 | 0,005 | 0,446 | 0,392 | 0,001 | 0,391 | 0,4 U/ml |
|   | 0,902 | 0,004 | 0,898 | 0,837 | 0,008 | 0,829 | 0,741 | 0,007 | 0,734 | 0,665 | 0,002 | 0,663 | 0,8 U/ml |
| 7 | 0,604 | 0,003 | 0,601 | 0,531 | 0,007 | 0,524 | 0,488 | 0,008 | 0,480 | 0,432 | 0,003 | 0,429 | 0,4 U/ml |
|   | 0,937 | 0,005 | 0,932 | 0,855 | 0,005 | 0,850 | 0,776 | 0,003 | 0,773 | 0,712 | 0,005 | 0,707 | 0,8 U/ml |
| 9 | 0,622 | 0,001 | 0,621 | 0,551 | 0,006 | 0,545 | 0,502 | 0,001 | 0,501 | 0,441 | 0,005 | 0,436 | 0,4 U/ml |
|   | 0,945 | 0,004 | 0,941 | 0,866 | 0,008 | 0,858 | 0,791 | 0,004 | 0,787 | 0,0702 | 0,006 | 0,696 | 0,8 U/ml |
|   | >1,5 | 0,006 |   | >1,5 | 0,007 |   | >1,5 | 0,003 |   | >1,5 | 0,004 |   | 4,5 U/ml |
| Kalbsserum inakti- viert als 50%ige Lsg. in 0,01M TRIS gepufferter Kochsalzlösung pH 7,2 keine Stabili- satorenzusätze | 0,502 | 0,005 | 0,497 | 0,226 | 0,004 | 0,222 | 0,097 | 0,001 | 0,096 |   |   |   | 0,4 U/ml |
|   | 0,813 | 0,007 | 0,806 | 0,385 | 0,005 | 0,382 | 0,162 | 0,002 | 0,160 |   |   |   | 0,8 U/ml |
|   | >1,5 | 0,004 |   | 0,882 | 0,005 | 0,877 | 0,553 | 0,002 | 0,551 |   |   |   | 4,5 U/ml |

**Patentansprüche**

**Patentansprüche für folgende Vertragsstaaten : AT, BE, CH, DE, FR, GB, GR, IT, LI, LU, NL, SE**

1. Stabilisiertes Peroxidasepräparat in Lösung, dadurch gekennzeichnet, daß es als Stabilisator hydrolysiertes Hühneralbumin oder eine Kombination aus Kalbsserum und Polyvinylpyrrolidon und/oder Calciumpropionat oder Gemische davon enthält.

2. Peroxidasepräparat nach Anspruch 1, dadurch gekennzeichnet, daß das hydrolysierte Hühneralbumin zusammen mit Kalbsserum vorliegt.

3. Peroxidasepräparat nach einem der vorstehenden Ansprüche, dadurch gekennzeichnet, daß es zusätzlich ein wasserlösliches Salz der 8-Anilino-1-naphthalinsulfonsäure enthält.

4. Peroxidasepräparat nach Anspruch 3, dadurch gekennzeichnet, daß das Salz ein Ammoniumsalz ist.

5. Peroxidasepräparat nach einem der vorstehenden Ansprüche, dadurch gekennzeichnet, daß es zusätzlich Methoxypolyethylenglycol enthält.

6. Peroxidasepräparat nach einem der vorstehenden Ansprüche, dadurch gekennzeichnet, daß die Peroxidase als Peroxidasekonjugat vorliegt.

7. Peroxidasepräparat nach Anspruch 6, dadurch gekennzeichnet, daß das Konjugat ein Peroxidase-Antikörperkonjugat ist.

8. Peroxidasepräparat nach einem der vorstehenden Ansprüche, dadurch gekennzeichnet, daß der oder die Stabilisator(en) in einer Konzentration von 0,1 bis 20 Gew.-%, bezogen auf die Präparatlösung, vorliegen.

9. Peroxidasepräparat nach einem der vorstehenden Ansprüche, dadurch gekennzeichnet, daß das Kalbsserum in einer Konzentration von 10 bis 75 Gew.-%, bezogen auf die Präparatlösung, vorliegt.

10. Testpackung zur Durchführung von enzymatischen Immuntests, enthaltend ein stabilisiertes Peroxidasepräparat nach einem der Ansprüche 1 bis 9.

**Patentansprüche für folgenden Vertragsstaat : ES**

1. Verfahren zur Herstellung eines stabilisierten Peroxydasepräparates in Lösung, dadurch gekennzeichnet, daß man dem Präparat als Stabilisator hydrolysiertes Hühneralbumin oder eine Kombination aus Kalbsserum und Polyvinylpyrrolidon und/oder Calciumpropionat oder Gemische davon hinzufügt.

2. Verfahren gemäß Anspruch 1, dadurch gekennzeichnet, daß als Stabilisator hydrolysiertes Hühneralbumin zusammen mit Kalbsserum verwendet wird.

3. Verfahren nach einem der vorstehenden Ansprüche, dadurch gekennzeichnet, daß man dem Präparat zusätzlich ein wasserlösliches Salz der 8-Anilino-1-naphthalinsulfonsäure beifügt.

4. Verfahren nach Anspruch 3, dadurch gekennzeichnet, daß man als Salz ein Ammoniumsalz verwendet.

5. Verfahren nach einem der vorstehenden Ansprüche, dadurch gekennzeichnet, daß man zusätzlich Methoxypolyethylenglycol beifügt.

6. Verfahren nach einem der vorstehenden Ansprüche, dadurch gekennzeichnet, daß als Peroxidase Peroxidasekonjugat eingesetzt wird.

7. Verfahren nach Anspruch 6, dadurch gekennzeichnet, daß als Konjugat ein Peroxidase-Antikörperkonjugat verwendet wird.

8. Verfahren nach einem der vorstehenden Ansprüche, dadurch gekennzeichnet, daß man den oder die Stabilisator(en) in einer Konzentration von 0,1 bis 20 Gew.-%, bezogen auf die Präparatlösung, verwendet.

9. Verfahren nach einem der vorstehenden Ansprüche, dadurch gekennzeichnet, daß man das Kalbsserum in einer Konzentration von 10 bis 75 Gew.-%, bezogen auf die Präparatlösung, verwendet.

10. Testpackung zur Durchführung von enzymatischen Immuntests, enthaltend ein stabilisiertes Peroxidasepräparat, hergestellt nach dem Verfahren gemäß einem der Ansprüche 1 bis 9.

**Claims**

**Claims for the following Contracting States : AT, BE, CH, DE, FR, GB, GR, IT, LI, LU, NL, SE**

1. Stabilized peroxidase preparation in solution, characterised in that it contains, as stabilizer, hydrolysed chicken egg albumin or a combination of calf's serum and polyvinyl pyrrolidone and/or calcium propionate or mixtures thereof.

2. Peroxidase preparation according to Claim 1, characterised in that the hydrolysed chicken egg albumin is present together with calf's serum.

3. Peroxidase preparation according to one of the preceding claims, characterised in that it contains, in addition, a water-soluble salt of 8-anilino-1-naphthalene sulphonic acid.

4. Peroxidase preparation according to Claim 3, characterised in that the salt is an ammonium salt.

5. Peroxidase preparation according to one of the preceding Claims, characterised in that it contains methoxypolyethylene glycol in addition.

6. Peroxidase preparation according to one of the preceding Claims, characterised in that the peroxidase is present as a peroxidase conjugate.

7. Peroxidase preparation according to claim 6, characterised in that the conjugate is a peroxidase antibody conjugate.

8. Peroxidase preparation according to one of the preceding Claims, characterised in that the stabilizer or stabilizers is or are present at a concentration of from 0.1 to 20% by weight, based on the solution of preparation.

9. Peroxidase preparation according to one of the preceding Claims, characterised in that the calf's serum is present at a concentration of from 10 to 75% by weight, based on the solution of preparation.

10. Test pack for carrying out enzymatic immune tests, containing a stabilized peroxidase preparation according to one of the Claims 1 to 9.

**Claims for the following Contracting State : ES**

1. A process for the preparation of a stabilized peroxidase preparation in solution, characterised in that hydrolysed chicken egg albumin or a combination of calf's serum and polyvinyl pyrrolidone and/or calcium propionate and/or mixtures thereof is/are added to the preparation as stabilizer.

2. A process according to Claim 1, characterised in that the stabilizer used is hydrolysed chicken egg albumin together with calf's serum.

3. A process according to one of the preceding Claims, characterised in that in addition, a water-soluble salt of 8-anilino-1-naphthalene sulphonic acid is added to the preparation.

4. A process according to Claim 3, characterised in that the salt used is an ammonium salt.

**5.** A process according to one of the preceding Claims, characterised in that methoxypolyethylene glycol is added in addition.

**6.** A process according to one of the preceding Claims, characterised in that the peroxidase used is a peroxidase conjugate.

**7.** A process according to Claim 6, characterised in that the conjugate used is a peroxidase antibody conjugate.

**8.** A process according to one of the preceding Claims, characterised in that the stabilizer or stabilizers is/are used at a concentration of from 0.1 to 20% by weight, based on the solution of preparation.

**9.** A process according to one of the preceding Claims, characterised in that the calf's serum is used at a concentration of from 10 to 75% by weight, based on the solution of preparation.

**10.** Test pack for carrying out enzymatic immune tests, containing a stabilized peroxidase preparation, prepared by the process according to one of the Claims 1 to 9.

**Revendications**

**Revendications pour les Etats contractants suivants : AT, BE, CH, DE, FR, GB, GR, IT, LI, LU, NL, SE**

**1.** Préparation de peroxydase stabilisée en solution, caractérisée en ce qu'elle contient comme stabilisateur de l'albumine de poule hydrolysable ou une combinaison de sérum de veau et de polyvinylpyrrolidone et/ou de propionate de calcium ou de mélanges de ces corps.

**2.** Préparation de peroxydase selon la revendication 1, caractérisée en ce que l'albumine de poule hydrolysée est présente avec du sérum de veau.

**3.** Préparation de peroxydase selon l'une des revendications précédentes, caractérisée en ce qu'elle contient en outre un sel soluble dans l'eau de l'acide 8-anilino-1-naphtalènesulfonique.

**4.** Préparation de peroxydase selon la revendication 3, caractérisée en ce que le sel est un sel d'ammonium.

**5.** Préparation de peroxydase selon l'une des revendications précédentes, caractérisée en ce qu'elle contient en outre du méthoxypolyéthylèneglycol.

**6.** Préparation de peroxydase selon l'une des revendications précédentes, caractérisée en ce que la peroxydase se présente sous forme de conjugué de peroxydase.

**7.** Préparation de peroxydase selon la revendication 6, caractérisée en ce que le conjugué est un conjugué peroxydase-anticorps.

**8.** Préparation de peroxydase selon l'une des revendications précédentes, caractérisée en ce que le ou les stabilisateur(s) est(sont) présent(s) à une concentration de 0,1 à 20% en poids, par rapport à la solution de préparation.

**9.** Préparation de peroxydase selon l'une des revendications précédentes, caractérisée en ce que le sérum de veau est présent à une concentration de 10 à 75% en poids par rapport à la solution de préparation.

**10.** Conditionnement expérimental pour tests immunologiques enzymatiques contenant une préparation de peroxydase stabilisée selon l'une des revendications 1 à 9.

**Revendications pour l'Etat contractant suivant : ES**

1. Procédé de préparation d'une préparation de peroxydase stabilisée en solution, caractérisé en ce qu'on ajoute à la préparation, comme stabilisateur, de l'albumine de poule hydrolysée ou une combinaison de sérum de veau et de polyvinylpyrrolidone et/ou de propionate de calcium ou de mélanges de ces corps.

2. Procédé selon la revendication 1, caractérisé en ce qu'on utilise comme stabilisateur de l'albumine de poule hydrolysée avec du sérum de veau.

3. Procédé selon l'une des revendications précédentes, caractérisé en ce qu'on ajoute en outre à la préparation un sel soluble dans l'eau de l'acide 8-anilino-1-naphtalènesulfonique.

4. Procédé selon la revendication 3, caractérisé en ce qu'on utilise comme sel un sel d'ammonium.

5. Procédé selon l'une des revendications précédentes, caractérisé en ce qu'on ajoute en outre du méthoxypolyéthylèneglycol.

6. Procédé selon l'une des revendications précédentes, caractérisé en ce qu'on utilise comme peroxydase un conjugué de peroxydase.

7. Procédé selon la revendication 6, caractérisé en ce qu'on utilise contre conjugué un conjugué peroxydase-anticorps.

8. Procédé selon l'une des revendications précédentes, caractérisé en ce qu'on utilise le ou les stabilisateur(s) à une concentration de 0,1 à 20% en poids, par rapport à la solution de préparation.

9. Procédé selon l'une des revendications précédentes, caractérisé en ce qu'on utilise le sérum de veau à une concentration de 10 à 75% en poids par rapport à la solution de préparation.

10. Conditionnement expérimental pour tests immunologiques enzymatiques contenant une préparation de peroxydase stabilisée préparée en suivant le procédé selon l'une des revendications 1 à 9.